# EUROPEAN PATENT APPLICATION

(11) **EP 3 617 181 A1**
(43) Date of publication of application: **04.03.2020**
(21) Application number: 18191761.8
(22) Date of filing: 30.08.2018
(51) Int. Cl.: C07C 67/14, C07C 67/347, C07C 69/618, C07C 69/74, C07C 209/56, C07C 211/40, C07C 231/02, C07C 233/58

(54) **SYNTHESIS OF TRANS-2-PHENYLCYCLOPROPYLAMINE OR A SALT OR SOLVATE THEREOF**

(71) Applicant: Arevipharma GmbH, 01445 Radebeul (DE)
(72) Inventor: BREUNING, Alexander, 01139 Dresden (DE); LIMMERT, Michael, 01277 Dresden (DE); DELLING, Axel, 01129 Dresden (DE); HOFMEIER, Harald, 01127 Dresden (DE); HULTSCH, Jana, 01689 Weinböhla (DE); MICKSCH, Maik, 01187 Dresden (DE); SOMMER, Christian, 01139 Dresden (DE)
(74) Representative: Prüfer & Partner mbB Patentanwälte · Rechtsanwälte

(57) **Abstract**

The present invention relates to the field of manufacturing trans-2-phenylcyclopropylamine or a salt or solvate thereof, in particular in the form of the sulphate salt. The obtained product, particularly when in the form of the synthesis of trans-2-phenylcyclopropylamine sulphate, is exceptionally pure, substantially free from reactants and reagents that are mutagenic, hazardous or of great concern to the health of humans or the environment.

## Description

The present invention relates to the field of manufacturing trans-2-phenylcyclopropylamine or a salt or solvate thereof, in particular the sulphate salt thereof. The present invention specifically relates to the synthesis of trans-2-phenylcyclopropylamine or its sulphate salt being substantially free from reactants and reagents that are mutagenic, hazardous or of great concern to the health of humans or the environment.

### Background prior art

Trans-2-phenylcyclopropylamine is a monoamine oxidase inhibitor, more specifically, trans-2-phenylcyclopropylamine acts as non-selective and irreversible inhibitor of the enzyme monoamine oxidase. It has been prepared normally in its sulphate salt form. It was first synthesized in 1948. It is used to treat major depressive disorders, including a typical depression, especially when there is an anxiety component.

According to a standard synthesis of trans-2-phenylcyclopropylamine or its salt diazoethylacetate is reacted with styrene to yield 2-phenyl cyclopropyl ethylcarboxylate, which, upon use of sodium azide, is transferred via a Curtius-rearrangement into the target compound trans-2-phenylcyclopropylamine or its salt, in particular its sulphate salt.

However, trans-2-phenylcyclopropylamine enantiomers have remarkably different pharmacological properties and inhibitory effects. For example, (+)-trans-2-phenylcyclopropylamine has a 15 to 20 times greater monoamine oxidase potency than the (-)-enantiomer. However, (-)-trans-2-phenylcyclopropylamine is a better inhibitor of noradrenaline and dopamine uptake than the (+)-enantiomer (A.S. Horn and S.H. Snyder, Journal of Pharmacological Experimental Therapy, 1972).

US 4,016,204 discloses a method for providing trans-2-phenylcyclopropylamine sulphate for improving the yield and purity of trans-2-phenylcyclopropane carboxylic acid. In particular, the intermediate trans-2-phenylcyclopropane carboxylic acid is obtained by reacting styrene with ethyldiazoacetate to form the ester, cis/trans-ethyl-2-phenylcyclopropane carboxylate, which is hydrolyzed to the acid and separated into its cis and transisomers by repeatedly recrystallizing the trans acid from hot water to form the trans-2-phenylcyclopropane carboxylic acid.

Accordingly, the known standard synthesis of trans-2-phenylcyclopropylamine sulphate can be summarized by the following scheme:

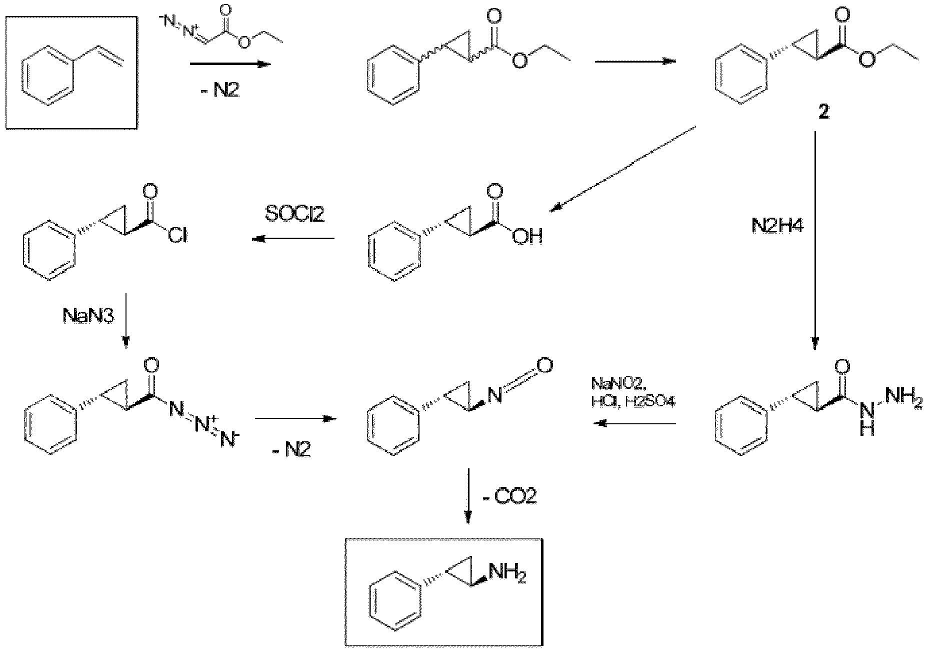

As can be derived from the reaction scheme the known roots of synthesis often apply commercially available hydroxybenzaldehyds, which were converted to the corresponding styrene. Further, the corresponding styrene were reacted with diazo compounds and resulted in poor stereo control of the obtained trans-2-phenylcyclopropyl carboxylic ester. Additionally, many gaseous byproducts were formed which resulted in a pressure built up in the reaction vessel which was hard to control. Besides the poor stereo control of about 65% of the desired trans diastereomer - together with its enantiomer - the used diazo compounds are toxic and hazardous. Furthermore, the use of sodium azide or hydrazine is very disadvantageously, as these materials are mutagenic or result in the formation of mutagenic intermediates (acyl azides or hydrazines).

However, other known single step procedures wherein the coupling of styrene with dibenzylformamide in the presence of MeTi(OiPr)₃ and an auxiliary Grignard compound resulted in diminished resolution of the pair of diastereomers due to no preference in the stereoisomers. Furthermore, titanium-mediated cyclopropanation which results in cyclopropylamines with an enantiomeric access of up to 84% has been disclosed. However, thereby elemental impurities are obtained due to the metal catalysts which places quite a burden on the workup in order to comply with ICH Q3D requirements for an active pharmaceutical ingredient.

Other processes for the preparation of aromatic cyclopropane esters and amides involve the reaction of styrene epoxide with a phosphonate in a Wadsworth type of reaction, whereby genotoxic reagents are applied (see WO 2008/018822).

Thus, although a large variety of synthetic procedures exists in the prior art, in all cases technological disadvantages can be found, may it be the use of Wittig type reagents that account for a significant purification effort, or sodium hydride, nitro-compounds, hydrazine, nitrous acid, sodium azide, heavy metals - as catalysts or reducing agents or as lewis acid and finally a poor stereo control. Thus, there is a great need for a synthesis of trans-2-phenylcyclopropylamine or its salt (in particular its sulphate salt) or its solvate which avoids the above listed drawbacks.

### Summary of the invention

In order to solve this problem, the present invention provides a process as defined in claim 1, and a process as defined in claim 12. Preferred embodiments are set forth in sub-claims and become apparent from the detailed description below.

Accordingly, the present invention provides an advantageous and efficient new route of synthesis which allows to obtain trans-2-phenylcyclopropylamine or a salt or solvate thereof, in particular the sulphate salt thereof, to be exceptionally pure and to avoid toxic or hazardous materials or any material that might account for a structural alert in terms of genotoxicity.

According to one aspect, the present invention provides a process for preparing a compound of formula (I) 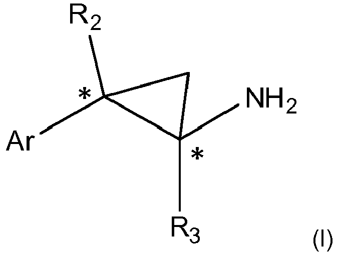 or a salt or solvate thereof, wherein each stereogenic center marked by * is in (R) or in (S) configuration, and
wherein R₂ and R₃ are identical or different, and are independently selected from
(i) hydrogen,
(ii) alkyl residues, optionally chiral, having from 1 to 12 carbon atoms, wherein the alkyl residues are optionally aryl and/or aryloxy substituted;
wherein Ar denotes respectively unsubstituted or substituted phenyl, heteroaryl, alkyl-aryl, alkoxy-aryl, preferably 2-pyridinyl or 4-pyridinyl, mono- or bicyclic, optionally substituted with methyl, ethyl, isopropyl, amino, hydroxy, methoxy, ethoxy, fluoro or chloro, most preferred Ar is unsubstituted phenyl;
the process comprising the steps of:
(a) providing a compound of formula (II) 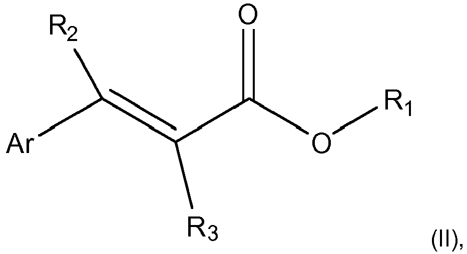 wherein Ar, R₂ and R₃ are defined as above, and R₁ is selected from
   (i) substituted or unsubstituted C₁-C₆ alkyl, preferably 2-propyl, 2-butyl
   (ii) substituted or unsubstituted C₃-C₆ cycloalkyl, preferably cyclohexyl
   (iii) substituted or unsubstituted C₆-C₁₈ alkyl-aryl groups, preferably C₆-C₁₈ alkyl-benzyl groups
   (iv) substituted or unsubstituted Si-groups, preferably Si-alkyl groups
(b) reacting the compound of formula (II) via a cyclopropanation reaction in a suitable solvent to obtain a compound according to formula (III), 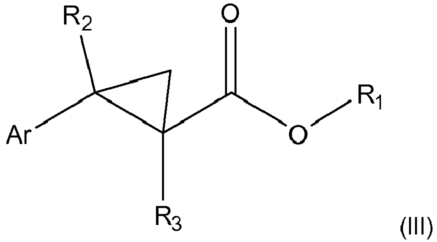 wherein Ar, R₁, R₂ and R₃ are defined as above,
(c) reacting the compound of formula (III) via an amidation reaction to obtain a compound according to formula (IV) 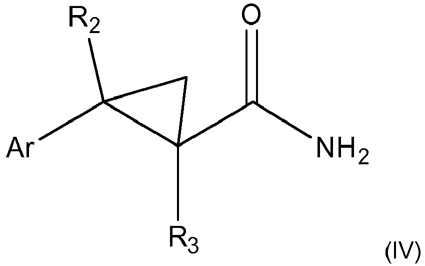 wherein Ar, R₁, R₂ and R₃ are defined as above,
(d) converting the compound of formula (IV) to the compound of formula (I),
(e) optionally forming a salt or solvate of the compound of formula (I).

In an embodiment of the present invention, the conversion of the compound of formula (IV) to the compound of formula (I) may be performed via a decarboxylation reaction, preferably via a Hofmann rearrangement, wherein more preferably the decarboxylation reaction is performed by using N-chloro succinic imide, N-bromo succinic imide, chlorine, bromine, or sodium hypochlorite and the decarboxylation reaction being carried out in presence of an organic solvent.

In an embodiment of the present invention the organic solvent is preferably selected from aliphatic alcohols such as ethanol, propanol, butanol, isopropanol, iso-butanol, tert-butanol or selected from halogenated solvents such as dichloromethane.

In an embodiment of the present invention the compound according to formula (I) is present in (1R, 2S)-Enantiomer, and/or in (1S, 2R)-Enantiomer.

In a preferred embodiment of the present invention R₂ and R₃ represent hydrogen and Ar represents unsubstituted phenyl.

According to a related aspect, the present invention provides a process for preparing tranylcypromine sulphate as defined by formula (Ia) 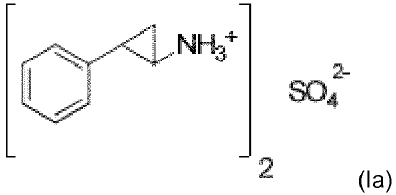 or solvate thereof, the process comprising:
(aa) addition of an aqueous solution of sulphuric acid to the compound of formula (Ib) 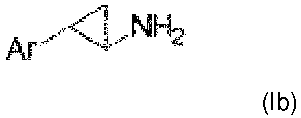 wherein Ar is unsubstituted phenyl,
(bb) obtaining tranylcypromine sulphate.

Step (bb) may include suitable synthetic or preparatory or purification step(s) to obtain tranylcypromine sulphate.

In a preferred embodiment of this related aspect, the compound of formula (Ib) had been obtained from a prior decarboxylation reaction by Hofmann rearrangement of the compound of formula 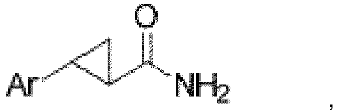 wherein Ar is unsubstituted phenyl,
whereafter the solution containing the intermediate product (Ib) has been purged by a combination of acidic/basic extraction sequences and ultimately the sulphate salt has been obtained by precipitation of the compound of formula (Ib) upon addition of the aqueous solution of sulphuric acid.

In a preferred embodiment of this related aspect, the compound of formula (Ib) was obtained by the process specified above and/or by any of the preferred embodiments thereof.

Preferably the prepared tranylcypromine sulphate of formula (Ia) has an HPLC purity of at least 99%, more preferably an HPLC purity of at least 99.5%, in particular an HPLC purity of at least 99.9%. More preferably, tranylcypromine sulphate is free from an any mutagenic impurities and is prepared without any mutagenic intermediates as demonstrated by Bacterial Reverse Mutation Tests according to OECD 471 and/or EU B.13/14 (see e.g. Example 6).

Within the present disclosure, the terms "*trans-2-phenylcyclopropylamine sulphate*" and "*tranylcypromine sulphate*" are used synonymous herein.

Further within the present disclosure, the term "*amidation reaction*" as used herein refers to any reaction or sequence of reactions, preferably sequence of chemical reactions, that lead or leads to an amide deriving from an ester.

Within the present disclosure, the term "salt" may refer typical salt formers of amine compounds, and may include, without being limited thereto, sulphate and hydrochloride. The term "sulphate salt" may mean hemisulphate salt.

Within the present disclosure, the term "solvate thereof" may refer to the compound trans-2-phenylcyclopropylamine or to the salt thereof, such as a solvate of the sulphate salt; the term "solvate" has its normal meaning.

### Detailed description of the invention

The process according to the present invention is advantageous and more efficient compared with prior art processes. It provides a synthesis that yields trans-2-phenylcyclopropylamine or a salt or solvate thereof, in particular the sulphate salt thereof, at extremely pure levels, and avoids toxic or hazardous materials or any material or intermediate that might account for a structural alert in terms of genotoxicity. It offers a quick, convenient, and experimentally simple preparation of suitably substituted cyclopropanes, with advantages over conventional procedures in the synthesis of trans-2-phenylcyclopropylamine or its salt (especially its sulphate salt) by ease of handling and relatively short reaction times.

The compound of formula (II) may be obtained from the compound of formula (V) 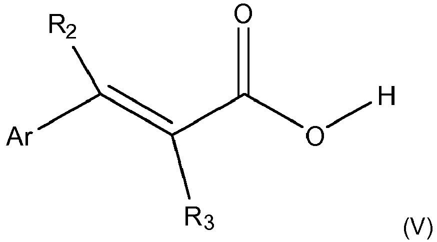 wherein Ar, R₁, R₂ and R₃ are defined as above,
by esterification according to standard procedures known to anyone skilled in the art, for instance by activation of the compound of formula (V) with thionyl chloride to give the respective acid chloride, which is converted into the compound of formula (II) by addition of or into an appropriate alcohol, optionally with the aid of a base.

In a preferred embodiment the compound of formula (II) is in the E or Z configuration, more preferably in the E configuration.

In a further preferred embodiment, the process for preparing a compound of formula (I) or a salt or solvate thereof, R₂ and R₃ are identical or different, and are independently selected from
(i) hydrogen,
(ii) alkyl residues, optionally chiral, having from 1 to 12 carbon atoms, wherein the alkyl residues are aryl and/or aryloxy substituted;
   wherein Ar denotes respectively unsubstituted or substituted phenyl, alkyl-aryl, alkoxy-aryl, 2-pyridinyl or 4-pyridinyl, mono- or bicyclic, preferably substituted with methyl, ethyl, isopropyl, amino, hydroxy, methoxy, ethoxy, fluoro or chloro, most preferred Ar is unsubstituted phenyl;
   wherein R₁ is selected from
   (i) substituted or unsubstituted C₁-C₆ alkyl, preferably C₃-C₆ alkyl, more preferably 2-propyl, 2-butyl
   (ii) substituted or unsubstituted C₃-C₆ cycloalkyl, preferably cyclohexyl.

In the most preferred embodiment of the present invention the compound of formula (I) is trans-2-phenylcyclopropylamine, and its most preferred pharmaceutically acceptable salt is trans-2-phenylcyclopropylamine sulphate.

In particular according to the present invention, the cyclopropanation reaction of a compound of formula (II) is trans-selective and leads to the compound of formula (III), which is obtained without use of a diazo compound. Accordingly, neither a genotoxic byproduct is build, nor a genotoxic reactant is used.

Preferably, the cyclopropanation reaction is performed:
(a) in the presence of a reagent, which is generated in situ from an ylide precursor and a base,
(b) more preferred the cyclopropanation reaction is performed in a polar, aprotic solvent,
(c) in particular preferred the cyclopropanation is performed for 20 minutes, preferably for 30 minutes, most preferably at least 1 h and at 20 °C, preferably 30 °C, most preferably at least 60 °C.

When in the preferred embodiments the cyclopropanation reaction is performed in case any or all of the conditions (a), (b) and/or (c) as listed above is/are met, the compound of formula (III) is obtained basically free from cis-configured diastereomer. In such preferred embodiments a quick, convenient and experimentally simple preparation of substituted cyclopropanes is possible, with advantages over conventional procedures: ease of handling (the mixture of a ylide precursor, e.g. sulfoxonium salt, and base can be stored indefinitely under anhydrous conditions without significant loss of activity) and reaction times are relatively short. Additionally, in case reaction step (c) is performed for at least 1 h and at least 60 °C a stable and controllable reaction procedure can be obtained in large-scale chemical production facilities.

More preferably, the ylide precursor according to a) is selected from R₃P=CR*R', R₂O⁺-C⁻R*R', R₃N⁺-C⁻R*R' or R₂S⁺-C⁻R*R', R₂S⁺(O)-C⁻R*R', more preferably selected from R₃P=CR*R', R₂S⁺-C⁻R*R', or R₂S⁺(O)-C⁻R*R', wherein R, R* and R' may be independently selected from
(i) hydrogen,
(ii) substituted or unsubstituted C₁-C₁₂ alkyl,
(iii) substituted or unsubstituted C₆-C₂₄ aryl,
(iv) substituted or unsubstituted C₇-C₂₄ alkylaryl,
(v) substituted or unsubstituted C₃-C₆ cycloalkyl,
(vi) substituted or unsubstituted C₁-C₁₈ alkoxy,
(vii) substituted or unsubstituted C₃-C₆ cycloalkoxy.

Preferably, the cyclopropanation reaction is performed in the presence of a sulphur ylide or a phosphorus ylide, more preferably a sulphur ylide, most preferred the ylide is a trialkyl-sulphonium oxide, particularly selected from dimethylsulfonium methylide, or dimethylsulfoxonium methylide.

The sulphur ylides may be prepared by deprotonation of the corresponding sulfonium salts which can be prepared from the reaction of either dimethylsulphide or dimethylsulphoxide with methyl iodide. The deprotonation may be performed via a base, for example selected from metal oxides, metal hydroxides, metal alcoholates, metal amides, wherein the metal may be selected from Li, Na, K, Rb, Cs, Fr, Be, Mg, Ca, Sr, Ba, Ra. The metal alcoholates may be selected from sodium methanolate, potassium methanolate, from sodium 2-methyl-2-propanolate or potassium 2-methyl-2-propanolate.

According to an embodiment of the present invention the compound of formula (III) is transferred into the compound of formula (IV) via transesterification, which preferably comprises the reaction of the compound of formula (III) with ammonia in the presence of a branched or linear alcohol, wherein the alcohol is preferably a primary alcohol, more preferred the alcohol is selected from methanol or ethanol, most preferred is methanol. According to a more preferred embodiment, the reaction occurs in the presence of a strong base, preferably the strong base is obtained by deprotonation of the branched or linear alcohol, more preferred the base is magnesium methanolate. Thereby, the amidation reaction occurs with a more reactive ester, which increases reaction speed and yield.

In another embodiment the amidation reaction further comprises an additive selected from Mg(OCH₃)₂, MgCl₂, CaCl₂, and CeCl₃, preferably CaCl₂. This increases the reaction speed, as the additive acts as a promoter.

In another embodiment of the present invention the compound of formula (III) undergoes saponification and subsequent activation with thionyl chloride or other known reagents to give the acid chloride, which upon addition of ammonia yields the desired compound of formula (IV). The beneficial effect of this embodiment is the use of inexpensive reagents, its quick and high yielding reaction steps and the obtainment of only one inorganic byproduct - ammonium chloride during amidation - which provides a good solubility can be easily removed by an aqueous work-up.

According to an embodiment of the present invention the conversion of the compound of formula (IV) to the compound of formula (I) is performed via a decarboxylation reaction, wherein the decarboxylation reaction is preferably performed by N-chloro succinic imide, N-bromo succinic imide, chlorine, bromine, or sodium hypochlorite and may be carried out in presence of an organic solvent.

Preferably the decarboxylation is achieved via Hofmann rearrangement. Thereby the use of hazardous chemicals, e.g. such as hydrazine in combination with nitrous acid or sodium azide as commonly employed, and the formation of intermediates with a structural alert in terms of genotoxicity, e.g. acyl azides or hydrazides is avoided.

According to a preferred embodiment of the present invention reagents that facilitate said Hofmann rearrangement are applied, such as for example N-bromo succinic amide (NBS), N-chloro succinic amide (NCS). In particular aqueous sodium hypochlorite solution, bromine or chlorine in presence of water and sodium hydroxide are applied.

Preferably, a sodium hypochlorite solution is applied as oxidizer for the compound of formula (IV) to be transferred into the non-isolated, labile carboxylic chloroamide of compound of formula (IV). Upon gentle heating it undergoes facile rearrangement into a non-isolated isocyanate intermediate which is hydrolysed by the water in the reaction mixture without delay to give the compound of formula (I).

The organic solvent according to a preferred embodiment may be selected from aliphatic alcohols such as ethanol, propanol, butanol, isopropanol, isobutanol, or may be selected from halogenated solvents such as dichloromethane, most preferably the solvent is isopropanol.

According to the present invention, optionally the compound of formula (I) is transferred to a salt or solvate thereof. Preferably, the compound of formula (I) is a pharmaceutically acceptable salt or solvate thereof.

In a further aspect, the present invention provides a process for preparing tranylcypromine sulphate according to formula (Ia) 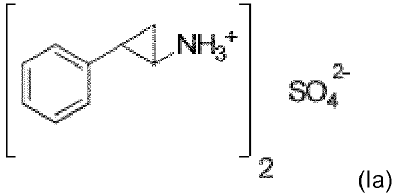 or a pharmaceutically acceptable salt or solvate thereof, the process comprising:
(aa) addition of an aqueous solution of sulphuric acid, preferably a diluted sulphuric acid,
more preferably a diluted sulphuric acid of 50:50 v/v, to the compound of formula (I) wherein R₂ and R₃ represent hydrogen and Ar is unsubstituted phenyl (i.e. selecting a compound of formula (Ib),
(bb) subsequently carrying out further synthetic step(s) to obtain tranylcypromine sulphate.

A beneficial effect that surprisingly arises from the use of the compound of formula (I), which was obtained according to the process disclosed according to this invention - and thus uniquely linking both aspects of the present invention - is that impurities, which may have formed during one of the previous process steps, are purged by acidic/basic extraction sequence and ultimately by precipitation of the compound of formula (I) upon addition of an acid of general formula HₙX or to obtain the salt or solvate of the compound of formula (I) as a pharmaceutically acceptable salt. In a preferred embodiment the acid is selected from hydrochloric acid (n = 1; X = Cl) or sulphuric acid (n =2; X = SO4). In an even more preferred embodiment of the present invention the acid is diluted aqueous acid, preferably diluted sulphuric acid. The diluted sulphuric acid has a concentration in water substantially less than normal concentrated sulfuric acid (having an azeotropic concentration of 98.3%), such as 80% v/v or below, preferably 60% v/v or below, more preferably 50% v/v or below. A particularly useful acid is diluted sulphuric acid diluted at about 50% v/v. The diluted hydrochloric acid has a concentration in water substantially less than normal concentrated hydrochloric acid (having an azeotropic concentration of 30% w/v), such as 25% w/v or below, preferably 20% w/v or below, more preferably 10% w/v or below, such as about 7% w/v.

Thus, by applying the compound of formula (I) obtained according to the process disclosed herein to the process of this embodiment, the overall process for obtaining the compound of formula (Ia) is much more controllable and - in addition thereto - by the prevention of hot spots the generation of further impurities due to thermal stress is minimized.

As regards the use of sulphuric acid for the salt formation: the procedure according to the present invention uses diluted sulphuric acid. Preferably diluted sulphuric acid may be derived from concentrated sulphuric acid which has been diluted with water, preferably with distilled water. More preferably, diluted sulphuric acid comprises concentrated sulphuric acid and water in a ratio between 20:80 to 80:20, more preferably the ratio is between 40:60 and 60:40, and more preferably the ratio is 50:50, i.e. 50%.

Accordingly, the precipitation may be performed in a suitable solvent or solvent mixtures such as toluene or an aliphatic alcohol, at a maximum temperature of 60 °C. In a preferred embodiment aqueous sulphuric acid is added to the compound of formula (I). Preferably, the compound of formula (I) is present in isopropanol with a toluene content of 0 - 50%.

The beneficial effect of the above mentioned use of sulphuric acid for the salt formation according to the present invention is the obtainment of essentially pure sulphate salt of the compound of formula (I). In particular, it contains less than 0.10% of related impurities by HPLC (gradient method with acetonitrile (mobile phase A) and phosphate buffer (mobile phase B); 50 - 95%; detection at 215nm). Accordingly, it was possible to obtain the prepared tranylcypromine sulphate of formula (Ia) has a surprising high purity, determined as an HPLC purity of at least 99%, more preferably at least 99.5% and even more preferably at least 99.9%.

Finally, according to an embodiment of the present invention a process for preparing a pharmaceutical composition comprising tranylcypromine sulphate or a pharmaceutically acceptable salt or solvate thereof is disclosed, wherein the process comprises the steps for obtaining tranylcypromine sulphate or a pharmaceutically acceptable salt or solvate thereof as disclosed herein, and mixing said obtained tranylcypromine sulphate or a pharmaceutically acceptable salt or solvate thereof, optionally with another active pharmaceutical ingredient, with a pharmaceutically acceptable excipient, carrier and/or diluent.

Thereby tranylcypromine sulphate or a pharmaceutically acceptable salt or solvate thereof is obtained in a very efficient, economically and resource friendly way.

### Examples

The invention will be illustrated in further detail with reference to examples which are merely illustrative and are not intended to limit the disclosure of the invention.

### Example 1 - E-Cinnamic acid isopropyl ester 1a (R = i-Pr)

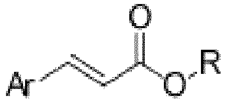

To a mixture of cinnamic acid (200 g) in toluene (300 mL) thionyl chloride (241 g) was added at 50 - 60 °C. After complete addition the reaction mixture was stirred for 6 h. After removal of toluene and residual thionyl chloride in vacuo isopropanol (830 mL) was added. The reaction mixture was stirred at reflux for 6 h. Isopropanol was removed in vacuo and toluene was added. The organic phase was washed 3 times with water basified with ammonia. Afterwards toluene was removed in vacuo resulting in 247 g of a yellow liquid (96%). HPLC purity: 99.8%, excluding toluene. ¹H NMR (500 MHz, DMSO-d6) δ7.73 - 7.68 (m, 2H), 7.63 (d, J = 16.0 Hz, 1H), 7.44 - 7.39 (m, 3H), 6.60 (d, J = 16.0 Hz, 1H), 5.02 (hept, J = 6.2 Hz, 1H), 1.26 (d, J = 6.3 Hz, 6H). ¹³C NMR (126 MHz, DMSO) δ= 165.7, 144.2, 134.0, 130.3, 128.8, 128.3, 118.5, 67.3, 21.7.

Other esters such as 1b (R = Et) and 1c (R = tBu) can be obtained via the same protocol.

### Example 2.1 - trans-2-Phenyl cyclopropyl isopropyl ester 2a (R = i-Pr)

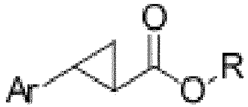

To a mixture of trimethylsulfoxonium iodide (92 g) and potassium-tert-butylate (38 g) DMSO (246 mL) was added. After stirring for 10 min the mixture was added to a solution of cinnamic acid isopropyl ester (65 g) in DMSO (154 mL) at 55 - 60 °C. After complete addition the reaction mixture was stirred for 1 h at 55 - 60 °C resulting in a mixture with a content of trans-2-phenyl cyclopropyl isopropyl ester in about 80%. The reaction mixture can be directly used for the saponification or can be worked up by extraction (vide infra).

### Example 2.2 - trans-2-Phenyl cyclopropyl isopropyl ester 2a (R = i-Pr)

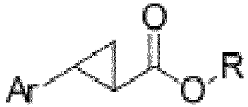

A solution of cinnamic acid isopropyl ester (2 g) in DMSO(5 mL) was added to a solution of sodium hydroxide (0.7 g) and trimethylsulfoxonium iodide (3.3 g) in DMSO (15 mL). After addition was finished, the reaction mixture was stirred 2 h at room temperature. The reaction was extracted four times with toluene. The combined organic phase was washed two times with water and evaporated to dryness. Yield: 1.3 g (59%); HPLC-Purity: 73%, yellowish oil.

### Example 2.3 - trans-2-Phenyl cyclopropane carboxylic acid ethyl ester 2b (R = Et)

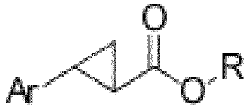

A solution of cinnamic acid ethyl ester (5 g) in DMSO (15 mL) was added to a cooled solution of potassium-tert-butylate (3.9 g) and trimethylsulfoxonium iodide (7.5 g) in DMSO (30 mL). After addition was finished, the mixture was heated to 50-60 °C and stirred for approx. 4 h at this temperature before it was extracted several times with ethyl acetate and brine. The organic phase was dried over anhydrous sodium sulphate and evaporated to dryness. Yield: 1.20 g (22%), yellowish oil.

### Example 2.4 - trans-2-Phenyl cyclopropane carboxylic acid tert-butyl ester 2c (R = tBu)

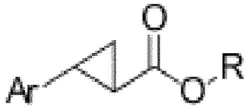

A solution of cinnamic acid tert-butyl ester (15 g) in DMSO (40 mL) was added to a cooled solution of potassium-tert-butylate (10 g) and trimethylsulfoxonium iodide (19.6 g) in DMSO (75 mL). The resulting mixture was stirred for approx.2 h at ambient temperature and subsequently extracted several times with ethyl acetate and brine. The organic phase was dried over anhydrous sodium sulphate and evaporated to dryness. Yield: 12.6 g (78%), brownish oil.

### Example 3.1 - trans-2-Phenylcyclopropane carboxamide via multi-step cascade

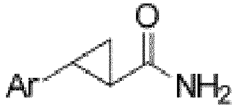

To the DMSO solution of trans-2-phenyl cyclopropyl isopropyl ester 2a of example 2.1 an aqueous solution of potassium hydroxide (45%, 45 mL) was added. The reaction mixture was stirred for 2 h at 60 °C. After addition of water and acidification with hydrochloric acid the reaction mixture was extracted four times with toluene. The toluene phases were combined and then extracted two times with water. Approximately 80% of the toluene was removed in vacuo. To the residue, containing the intermediate carboxylic acid, thionyl chloride (51 g) was added at 60 °C. After complete addition the reaction mixture was stirred for 2 h at 60 °C. After removal of toluene and residual thionyl chloride in vacuo tetrahydrofurane (110 mL) was added. Gaseous ammonia was added into the reaction mixture, containing the intermediate carboxylic acid chloride, until said carboxylic acid chloride was essentially fully consumed. Tetrahydrofurane (87 mL) and ethyl acetate (31 mL) were added and the reaction mixture was extracted five times with water. The solvent was removed in vacuo and the residue, containing the carboxamide, was recrystallized from isopropanol to obtain 29 g of the title compound as a colourless to slight brown solid (55% based on starting material 1a) with a HPLC purity of 99.8%. ¹H NMR (500 MHz, DMSO-d6) δ7.56 (s, 1H), 7.26 (t, J = 7.5 Hz, 2H), 7.17 (t, J= 7.3 Hz, 1H), 7.11 (d, J = 7.8 Hz, 2H), 6.87 (s, 1H), 2.21 (dt, J = 9.5, 5.8 Hz, 1H), 1.82 (dt, J = 8.9, 4.8 Hz, 1H), 1.32 (dt, J = 9.1, 4.6 Hz, 1H),1.21 - 1.13 (m, 1H). ¹³C NMR (126 MHz, DMSO) δ= 172.9, 141.2, 128.3, 125.8, 125.7, 25.4, 23.9, 15.2.

### Example 3.2 - trans-2-Phenylcyclopropane carboxamide via transesterification and amidation

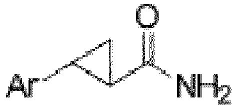

A pressure tube was charged with the trans-2-phenylcyclopropyl isopropyl ester of example 2.1 (3 mmol), ammonia in methanol (5 mL, 6-7 M) and magnesium methanolate in methanol (5 mL, 0.6 M). The reaction mixture was stirred for approx. 24 h at 80 °C, cooled and filtered. The filtrate was partially evaporated and extracted with 50 mL ethyl acetate and 50 mL potable water (slightly acidified at pH 4). The organic phase was dried over anhydrous sodium sulphate and evaporated to dryness. Yield: 3.90 g (75%, purity: 89%), brownish solid.

### Example 4 - trans-2-phenylcyclopropylamine

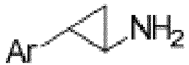

A cooled mixture of trans-2-phenylcyclopropane carboxamide (36 g), aqueous sodium hydroxide (50%, 18 mL), isopropanol (76 mL) and aqueous sodium hypochlorite (9.5%, 366 g) was stirred for 2 h. Afterwards aqueous sodium hydroxide (50%, 88 mL) was added and the reaction mixture was stirred at reflux for 1 h. Toluene (237 mL) was added and the reaction mixture was filtrated. The phases were separated and the organic phase was extracted two times with water (pH 10 - 11). To the organic phase water (355 mL) was added an acidified to a pH value of 2 - 3. The phases are separated and the aqueous phase was extracted with toluene two times. Toluene (237 mL) was added to the aqueous phase and basified to a pH value of 10 - 11. The phases were separated and the organic phase was extracted two times with water (pH 10- 11). Afterwards toluene was removed in vacuo. This concentrated product solution can be used *in situ* for further processing (vide infra). If the solvent was removed trans-2-phenylcyclopropylamine can be isolated as a lightly yellow liquor (68%). HPLC purity: 99.7% (residual toluene not included).

### Example 5 - Tranylcypromine sulphate

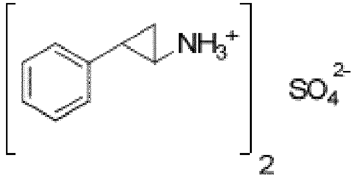

A solution of trans-2-phenylcyclopropylamine (16 g) in isopropanol (47 mL) was heated to 40 °C. An aqueous solution of sulphuric acid (50%) was added at 40 - 50 °C until a pH value of 2.5 - 3.5 was obtained. Afterwards the reaction mixture was stirred at 60 °C for 30 min and cooled to 0 - 5 °C. The precipitate was isolated by filtration and washed with isopropanol. Tranylcypromine sulphate was obtained as a colourless solid in 64% yield (based on trans-2-phenylcyclopropane carboxamide) with an HPLC purity of >99.9%. ¹H NMR (500 MHz, D2O) δ7.44 (t, J = 7.6 Hz, 2H), 7.36 (t, J = 7.4 Hz, 1H), 7.28 (d, J = 7.3 Hz, 2H), 2.95 (dt, J = 7.9, 4.0 Hz, 1H), 2.52 (ddd, J = 10.2, 6.6, 3.6 Hz, 1H), 1.52 (ddd, J = 10.7, 6.9, 4.5 Hz, 1H), 1.41 (q, J = 6.9 Hz, 1H). NH protons not detected due to H/D exchange; ¹³C NMR (126 MHz, D2O) δ= 138.8, 128.9, 127.0, 126.4, 30.7, 20.9, 12.6.

### Example 6 - Result of test on mutagenicity ("AMES-Test")

The mutagenic potential of the intermediates
i) trans-2-Phenyl cyclopropyl isopropyl ester 1a,
ii) trans-2-Phenyl cyclopropyl carboxylic acid,
iii) trans-2-Phenyl cyclopropyl carboxylic acid chloride and
iv) trans-2-Phenyl cyclopropane carboxamide
with the Bacterial Reverse Mutation Test following OECD 471 and EU B.13/14 were determined. The bacterial reverse mutation test is commonly employed as an initial screen for genotoxic activity and, in particular, for point mutation-inducing activity.

Principle of the test method: Suspensions of bacterial cells were exposed to the test substance in the presence and in the absence of an exogenous metabolic activation system. In the plate incorporation method, these suspensions were mixed with an overlay agar and plated immediately onto minimal medium. In the pre-incubation method, the treatment mixture was incubated and then mixed with an overlay agar before plating onto minimal medium. For both techniques, after 2 or 3 days of incubation, revertant colonies were counted and compared to the number of spontaneous revertant colonies on solvent control plates.

Bacterial Reverse Mutation Test using five strains of Salmonella typhimurium. The studies demonstrated that the investigated intermediates are not mutagenic in the tested *Salmonella typhimurium* strains.

## Claims

1. A process for preparing a compound of formula (I) 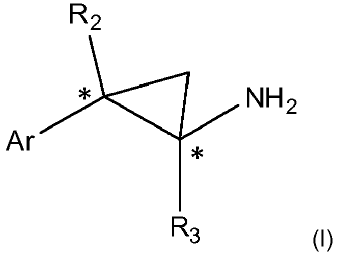 or a salt or solvate thereof, wherein each stereogenic center marked by * is in (R) or in (S) configuration, and
wherein R₂ and R₃ are identical or different, and are independently selected from
(i) hydrogen,
(ii) alkyl residues, optionally chiral, having from 1 to 12 carbon atoms, wherein the alkyl residues are optionally aryl and/or aryloxy substituted;
wherein Ar denotes respectively unsubstituted or substituted phenyl, heteroaryl, alkyl-aryl, alkoxy-aryl;
the process comprising the steps of:
(a) providing a compound of formula (II) 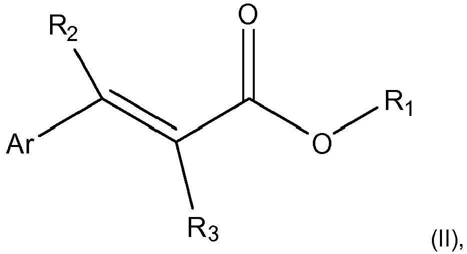 wherein Ar, R₂ and R₃ are defined as above, and R₁ is selected from
(i) substituted or unsubstituted C₁-C₆ alkyl,
(ii) substituted or unsubstituted C₃-C₆ cycloalkyl,
(iii) substituted or unsubstituted C₆-C₁₈ alkyl-aryl groups,
(iv) substituted or unsubstituted Si-groups,
(b) reacting the compound of formula (II) via a cyclopropanation reaction in an organic solvent to obtain a compound according to formula (III), 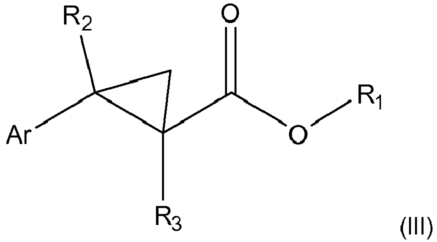 wherein Ar, R₁, R₂ and R₃ are defined as above,
(c) reacting the compound of formula (III) via an amidation reaction to obtain a compound according to formula (IV) 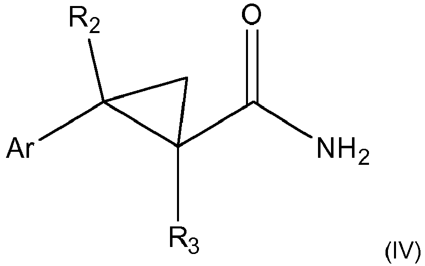 wherein Ar, R₁, R₂ and R₃ are defined as above,
(d) converting the compound of formula (IV) to the compound of formula (I),
(e) optionally forming a salt or solvate of the compound of formula (I).

2. The process according to claim 1, wherein the conversion of the compound of formula (IV) to the compound of formula (I) is performed via a decarboxylation reaction, preferably via a Hofmann rearrangement, wherein more preferably the decarboxylation reaction is performed by using N-chloro succinic imide, N-bromo succinic imide, chlorine, bromine, or sodium hypochlorite and is carried out in presence of an organic solvent, in particular wherein the organic solvent is selected from aliphatic alcohols or selected from halogenated solvents such as dichloromethane, preferably the solvent is isopropanol.

3. The process according to any one of the preceding claims, wherein compound according to formula (I) is present as (1R, 2S)-Enantiomer, and/or as (1S, 2R)-Enantiomer, and/or wherein the exocyclic double bond of the compound of formula (II) is in the E- or Z-configuration or a mixture thereof, preferably in E-configuration.

4. The process according to any one of the preceding claims, wherein R₂ and/or R₃ represent hydrogen, preferably R₂ and R₃ both represent hydrogen; and/or whereon Ar represent unsubstituted phenyl.

5. The process according to any one of the preceding claims, wherein the amidation reaction is performed with ammonia in the presence of a branched or linear alcohol, and preferably a strong base, more preferably the strong base is magnesium methanolate; the alcohol is preferably a primary alcohol, more preferably the alcohol is selected from methanol or ethanol, most preferred is methanol.

6. The process according to claim 5, wherein the amidation reaction further comprises an additive selected from Mg(OCH₃)₂, MgCl₂, CaCl₂, and CeCl₃.

7. The process according to any of the preceding claims, wherein the amidation reaction comprises a transesterification reaction.

8. The process according to any one of the preceding claims, wherein the compound of formula (II) is obtained by esterification of a compound of formula (V) 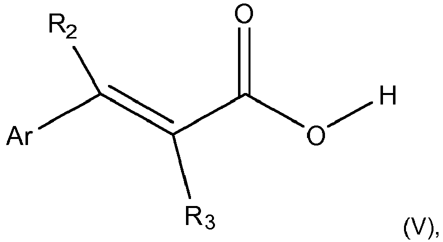 wherein Ar, R₂ and R₃ are defined as above.

9. The process according to any one of the preceding claims, wherein the cyclopropanation reaction is performed in the presence of an ylide, preferably prepared from a ylide precursor selected from R₃P=CR*R', R₂O⁺-C⁻R*R', RsN⁺-C⁻R*R' or R₂S⁺-C⁻R*R', R₂S⁺(O)-C⁻R*R', more preferably selected from R₃P=CR*R', R₂S⁺-C⁻R*R', or R₂S⁺(O)-C⁻R*R', wherein R, R* and R' are independently selected from
(i) hydrogen,
(ii) substituted or unsubstituted C₁-C₁₂ alkyl,
(iii) substituted or unsubstituted C₆-C₂₄ aryl,
(iv) substituted or unsubstituted C₇-C₂₄ alkylaryl,
(v) substituted or unsubstituted C₃-C₆ cycloalkyl,
(vi) substituted or unsubstituted C₁-C₁₈ alkoxy,
(vii) substituted or unsubstituted C₃-C₆ cycloalkoxy,

10. The process according to claim 9, wherein the ylide is a trialkyl-sulphonium oxide, preferably the ylide is selected from dimethylsulfonium methylide, or dimethylsulfoxonium methylide.

11. The process according to any one of the preceding claims, wherein the organic solvent used for the cyclopropanation reaction is a polar, aprotic solvent.

12. A process for preparing tranylcypromine sulphate as defined by formula (Ia) 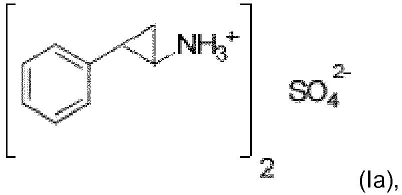 or solvate thereof, the process comprising:
(aa) addition of an aqueous solution of sulphuric acid to the compound of formula (Ib) 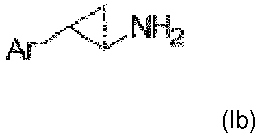 wherein Ar is unsubstituted phenyl,
(bb) obtaining tranylcypromine sulphate.

13. The process according to claim 12, wherein the compound of formula (Ib) is obtained from a prior decarboxylation reaction by Hofmann rearrangement of the compound of formula 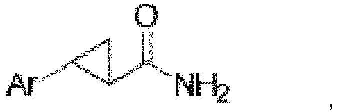
wherein Ar is unsubstituted phenyl,
whereafter the solution containing the intermediate product (Ib) is purged by a combination of acidic/basic extraction sequences.

14. The process according to claim 12 or 13, wherein step (bb) comprises precipitation of the compound of formula (Ib) upon addition of the aqueous solution of sulphuric acid, preferably wherein the prepared tranylcypromine sulphate of formula (Ia) has an HPLC purity of at least 99%, more preferably an HPLC purity of at least 99.5%, in particular an HPLC purity of at least 99.9%; and/or preferably wherein the prepared tranylcypromine sulphate of formula (Ia) is free from an any mutagenic impurities and is prepared without any mutagenic intermediates as demonstrated by Bacterial Reverse Mutation Tests according to OECD 471 and/or EU B.13/14.

15. A process for preparing a pharmaceutical composition comprising tranylcypromine sulphate or a pharmaceutically acceptable salt or solvate thereof, the process comprising the steps of carrying out the process according to anyone of claims 1 - 14 to obtain tranylcypromine or the corresponding pharmaceutically acceptable salt or solvate thereof, and mixing said obtained tranylcypromine or a pharmaceutically acceptable salt or solvate thereof, optionally with another active pharmaceutical ingredient, with a pharmaceutically acceptable excipient, carrier and/or diluent.
